# EUROPEAN PATENT SPECIFICATION

(11) **EP 2 740 727 B1**
(45) Date of publication and mention of the grant of the patent: **22.10.2014**
(21) Application number: 12196113.0
(22) Date of filing: 07.12.2012
(51) Int. Cl.: C07D 239/95, A61K 31/517, A61P 25/08

(54) **Quinazolinone analogues for use as anticonvulsant agents**
Chinazolinanaloga zur Verwendung als Antikrampfmittel
Analogues de quinazolinone destinés à être utilisés en tant qu'agents anticonvulsivants

(43) Date of publication of application: 11.06.2014
(73) Proprietor: King Saud University, 11421 Riyadh (SA)
(72) Inventor: Al-Salem, Huda S.A., Riyadh 11333 (SA); El-Subbagh, Hussein I., Riyadh 11333 (SA); El-Taher, Kamal E.H., Riyadh 11333 (SA); Hegazy, Gehan H., Riyadh 11333 (SA); Al-Obaid, Abdulrahman M., Riyadh 11333 (SA)
(74) Representative: Goddar, Heinz J.

(56) References cited:
- GURSOY A ET AL: "Synthesis and primary cytotoxicity evaluation of 3-[[(3-phenyl-4(3H)-quinazolinone-2-yl)mer captoacetyl]hydrazono]-1H-2-indoli nones", EUROPEAN JOURNAL OF MEDICINAL CHEMISTRY, EDITIONS SCIENTIFIQUE ELSEVIER, PARIS, FR, vol. 38, no. 6, 1 June 2003 (2003-06-01), pages 633-643, XP004433584, ISSN: 0223-5234, DOI: 10.1016/S0223-5234(03)00085-0
- KARALI, NILGUN ET AL: "New cyclohexylidenehydrazide and 4-aza-1-thiaspiro[4.5]decan-3-one derivatives of 3-phenyl-4(3H)-quinazolinones", FARMACO , 53(5), 346-349 CODEN: FRMCE8; ISSN: 0014-827X, vol. 53, 1998, pages 348-349, XP002690899,
- DATABASE CAPLUS [Online] CHEMICAL ABSTRACTS SERVICE, COLUMBUS, OHIO, US; MAHMOUD, MAHMOUD R. ET AL: "Synthesis and spectral characterisation of novel 2,3-disubstituted quinazolin-4(3H)-one derivatives", XP002690900, retrieved from STN Database accession no. 2012:492815 & MAHMOUD, MAHMOUD R. ET AL: "Synthesis and spectral characterisation of novel 2,3-disubstituted quinazolin-4(3H)-one derivatives", JOURNAL OF CHEMICAL RESEARCH , 36(2), 66-71 CODEN: JCROA4; ISSN: 1747-5198, 2012,
- DATABASE CAPLUS [Online] CHEMICAL ABSTRACTS SERVICE, COLUMBUS, OHIO, US; GHORAB, MUSTAFA M. ET AL: "Synthesis and insecticidal activity of some new 3-[4(3H)-quinazolinone-2-yl)thiomethyl]-1, 2,4-triazole-5-thiols", XP002690901, retrieved from STN Database accession no. 1996:546937 & GHORAB, MUSTAFA M. ET AL: "Synthesis and insecticidal activity of some new 3-[4(3H)-quinazolinone-2-yl)thiomethyl]-1, 2,4-triazole-5-thiols", PESTICIDE SCIENCE , 48(1), 31-35 CODEN: PSSCBG; ISSN: 0031-613X, 1996,
- DATABASE CAPLUS [Online] CHEMICAL ABSTRACTS SERVICE, COLUMBUS, OHIO, US; GUERSOY, AYSEL ET AL: "Synthesis and anticonvulsant activity of new acylthiosemicarbazides and thiazolidones", XP002690902, retrieved from STN Database accession no. 1996:80424 & GUERSOY, AYSEL ET AL: "Synthesis and anticonvulsant activity of new acylthiosemicarbazides and thiazolidones", FARMACO , 50(12), 857-66 CODEN: FRMCE8, 1995,
- DATABASE CAPLUS [Online] CHEMICAL ABSTRACTS SERVICE, COLUMBUS, OHIO, US; AL-ASHMAWI, MOHAMED I. ET AL: "Synthesis of certain substituted 7-chloro-4-quinazolones of pharmaceutical interest", XP002690903, retrieved from STN Database accession no. 1994:483267 & AL-ASHMAWI, MOHAMED I. ET AL: "Synthesis of certain substituted 7-chloro-4-quinazolones of pharmaceutical interest", ZAGAZIG JOURNAL OF PHARMACEUTICAL SCIENCES , 2(2), 158-68 CODEN: ZJPSEV; ISSN: 1110-5089, 1993,
- DATABASE CAPLUS [Online] CHEMICAL ABSTRACTS SERVICE, COLUMBUS, OHIO, US; ABDEL HAMID, SAMI G. ET AL: "Synthesis and anticonvulsant activity of some new 4-Oxo-3H-quinazoline analogs", XP002690904, retrieved from STN Database accession no. 2001:501539 & ABDEL HAMID, SAMI G. ET AL: "Synthesis and anticonvulsant activity of some new 4-Oxo-3H-quinazoline analogs", MEDICINAL CHEMISTRY RESEARCH , 10(6), 378-389 CODEN: MCREEB; ISSN: 1054-2523, 2001,
- PRAVEEN KUMAR ET AL: "Design, synthesis and potential 6Hz psychomotor seizure test activity of some novel 2-(substituted)-3-{[substituted]amino}quin azolin-4(3)-one", EUROPEAN JOURNAL OF MEDICINAL CHEMISTRY, EDITIONS SCIENTIFIQUE ELSEVIER, PARIS, FR, vol. 46, no. 4, 10 January 2011 (2011-01-10), pages 1006-1018, XP028185748, ISSN: 0223-5234, DOI: 10.1016/J.EJMECH.2011.01.009 [retrieved on 2011-01-15]

## Description

The present invention relates to quinazolinone compounds useful as anticonvulsant agents, pharmaceutical composition containing the compounds as well as methods for their preparation.

Epilepsy is one of the most common neurological disorders, which is characterized by seizures that take various forms and result from episodic neural discharges. The forms of seizures depend on the part of the brain affected. Epilepsy affects 0.5-1% of the population. Although there is no recognizable cause which can be identified, patient may develop seizure after brain trauma, infection or tumor growth, or other kinds of neurological diseases, including various inherited neurological syndromes. Epilepsy is treated mainly with drugs, though brain surgery may be used for severe cases. Current antiepileptic drugs are effective in controlling seizures in about 70% of patients, but their use is often limited by side-effects.

GABA (y-aminobutyric acid) is the principal inhibitory transmitter of many CNS pathways regulating numerous neurological functions including convulsions, anxiety and sleep activity. GABA acts on the GABA_{A} chloride ion channel. Molecular biology studies have demonstrated that several different receptor subunits combine to form the GABA_{A} receptor complex with a number of receptor subtypes such as benzodiazepine receptor (BzR). Ligands acting at the BzR allosteric binding site of the GABA_{A} receptor modulate the action of GABA on chloride ion flux. They show a wide variety of pharmacological actions ranging from full agonistic effects such as sedative/hypnotic, anxiolytic and anticonvulsant activities; to inverse agonistic effects such as anxiogenic, and proconvulsant activities. Several classes of chemical compounds such as benzodiazepines, steroids and barbiturates bind to the GABA_{A} chloride ion channel complex. The azepine derivatives such as imidazo[2,1-*b*]thiazepine exhibited affinity for BzR. Their profile of action was characterized as partially agonistic at the BzRs on the basis of the GABA shift.

The α-Amino-3-hydroxy-5-methyl-4-isoxazole propionate receptors (AMPARs) are glutamate-gated ion channels which mediate the majority of fast excitatory synaptic transmissions in the mammalian brain. Hyperactivation of AMPARs is implicated in a broad range of acute and chronic neurodegenerative and neuropsychiatric conditions such as epilepsy. Thus, the modulation of AMPARs as potential targets for therapeutic intervention in many neurological disorders is one of the goals in neuropharmacology (Dingledine R. et al. Pharmacol. Rev. 1999, 51, 7-61; Lees G. J., Drugs 2000, 59, 33-78). Consequently, several antagonists of AMPAR such as talampanel (Rogawski M. A. Trends Pharmacol. Sci. 1993, 14, 325-331) and CFM-2 (Quartarone S., et al. IL Farmaco 2004, 59, 353-358) have been reported and showed promising therapeutic potential for the prevention and treatment of epilepsy.

Drawbacks of anticonvulsants used in the prior art are numerous starting from mild sedation to significant types of toxicity. The sedative-hypnotic effect is very common which markedly affects the cognitive function with behavioral deficits, abnormal visual acuity and reduced contrast sensitivity, optimism, severe blood disorders and liver damage. Moreover, known anticonvulsants are among the most common causes of fetal malformations. There are reports of patients developing reversible cognitive decline or reversible Parkinsonism, often both. Common imaging findings among these reports are reversible cortical pseudo-atrophy and enlargement of the lateral ventricles. Epilepsy is resistant to drug treatment in about one-third of the cases.

It is an object of the present invention to provide anticonvulsant agents which overcome the drawbacks of the prior art. Especially compounds shall be provided exhibiting potent *in vivo* anticonvulsant activity.

This object is achieved by a compound according to formula 1: 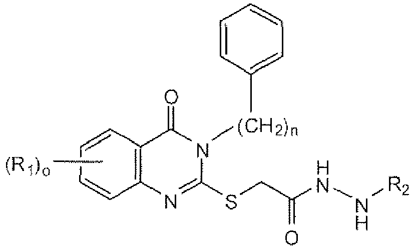 wherein each R₁ is independently selected from the group consisting of chloro, methoxy, aryl, preferably phenyl or naphthyl, heteroaryl, preferably furyl, pyrrolyl, thienyl, imidazolyl, thiadiazolyl, pyridinyl, pyridazinyl, pyrimidinyl, benzothiazolyl and oxadiazolyl, mercapto and alkylthio, most preferably is chloro or methoxy; R₂ is -CO-(CH₂)ₘ-R₃; R₃ is selected from the group consisting of alkyl, alkoxyl, aryl, or heteroaryl ring system, preferably furyl, pyrrolyl, thienyl, imidazolyl, thiadiazolyl, pyridinyl, pyridazinyl, pyrimidinyl, benzothiazolyl or oxadiazolyl; o is an integer from 1-4; n and m are independently selected integers form 0-10, preferably from 0-5.

Besides to the neutral, uncharged form, presented by the general formula 1, the inventive compound can be present in form of its addition salt, preferably selected from hydrochloride, hydrobromide, phosphate, nitrate, acetate, malate, succinate, fumarate, tartrate, salicylate, sorbate, lactate, p-toluene sulphate, or naplithalene-1,5-disulionate salts. The addition salts of compounds presented by general formula 1 are also a solution to the object underlying the present invention just like the neutral, uncharged compounds, presented by the general formula 1.

The object is further achieved by a method for preparing the inventive compound, according to the following reaction sequence: 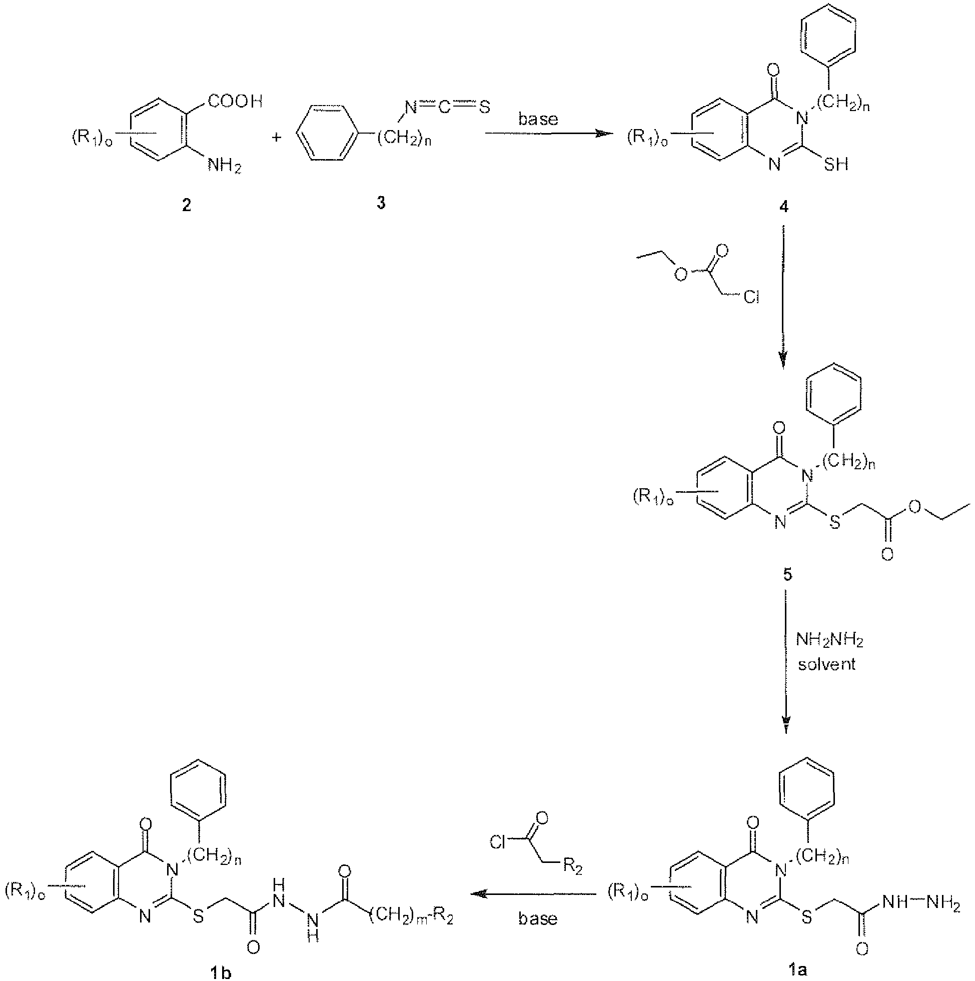 wherein R₁, R₂, n, m and o are defined as in claim 1.

Compound 1b as well as its precursor compound 1a are inventive compounds of the general formula 1.

The object is also achieved by a pharmaceutical composition comprising at least one of the inventive compounds.

Moreover, the object is achieved by the inventive compound for use in the treatment of convulsion and/or epilepsy.

Finally, the object is achieved by the inventive pharmaceutical composition for use in the treatment of convulsion and/or epilepsy.

Surprisingly, it was found that the compounds according the present invention solve the problem underlying the present invention by possessing anticonvulsant activity superior over the activity of compounds known in the prior art. In particular, it was found that compounds according to the present invention exhibit an activity 3-10 fold higher than that of valproate sodium which is used as a standard anticonvulsant agent in treatment of epilepsy.

The term "alkyl" in terms of the present invention is to be understood to comprise linear and branched alkyl groups. Preferably, alkyl groups in terms of the present invention are alkyl groups having 1 to 20 carbon atoms, more preferably 1 to 10 carbon atoms, most preferably 1 to 5 carbon atoms. The term "halo" shall comprise derivatives which are mono-, di-, tri- or poly-halosubstituted.

If possible, all substituents R₁ and R₃ may be further substituted, for example by halogen, amino, substituted amino, alkyl, haloalkyl, alkoxy or haloalkoxy, alkylamino, arylthio, heteroarylthio, arylamino or a ring system, wherein the ring system is preferably furyl, pyrrolyl, thienyl, imidazolyl, thiadiazolyl, pyridinyl, pyridazinyl, pyrimidinyl, benzothiazolyl and oxadiazolyl.

The compounds of the invention and their analogues (**1**) are synthesized according to the inventive method according to Scheme 1. A proper substituted-anthranilic acid (**2**) is reacted with a suitable isothiocyanate derivative (**3**) in presence of a base, such as triethylamine or piperidine, in a suitable solvent, such as, for example, ethanol, at temperatures ranging from 100-120°C. The product (**4**) can be purified by general known purification methods, such as silica gel or neutral alumina chromatography. Compounds of the formula (**4**) are reacted with ethyl chloroacetate in a suitable solvent, such as acetone or acetonitrile, at temperatures ranging from room temperature to 100°C, to produce products of the formula (**5**). Compounds of the formula (**5**) are reacted with hydrazine hydrate to give compounds of the formula (6). Compounds of the formula 1 can be obtained by the reaction of compounds of the formula (5) with hydrazine hydrate to give compounds of the formula (**1a**). The hydrazones (**1a**) can then be reacted with a variety of acid chlorides to produce compounds (**1b**). The product of the formula 1 (meaning both the compounds of groups **1a** and **1b**) can be purified by general known purification means, such as silica gel or neutral alumina chromatography.

Compounds of the invention, according to formula 1, and their acid addition salts display anticonvulsant activity. The present invention includes pharmaceutical formulations which, in addition to non-toxic, inert pharmaceutically suitable excipients, contain one or more active compounds according to the invention, or which consist of one or more active compounds according to the invention, as well as processes for the preparation of these formulations.

The present invention also includes pharmaceutical formulations in dosage units. This means that the formulations are in the form of individual parts, for example tablets, dragees, capsules, pills, and ampoules, of which the content of active compound corresponds to a fraction or a multiple of an individual dose. The dosage units can contain, for example, 1, 2, 3 or 4 individual doses or 1/2, 1/3 or 1/4 of an individual dose. An individual dose preferably contains the amount of active compound which is given in one administration and which usually corresponds to a whole, a half, a third or a quarter of a daily dose.

By non-toxic, inert pharmaceutically suitable excipients there are to be understood solid, semi-solid or liquid diluents, fillers and formulations auxiliaries of every kind.

Tablets, dragees, capsules, pills, granules, solutions and sprays may be mentioned as preferred pharmaceutical formulations.

Tablets, dragees, capsules and pills can contain the active compound or compounds alongside the customary excipients, such as (a) fillers and extenders, for example starches, lactosc, sucrose, glucose, mannitol and silica, (b) binders, for example carboxymethylcellulose, alginates, gelatin and polyvinylpyrrolidone, (c) humectants, for example agar-agar, calcium carbonate and sodium bicarbonate, (e) solution retarders, for example paraffin, and (f) resorption accelerators, for example quaternary ammonium compounds, (g) wetting agents, for example cetyl alcohol and glycerol monostearate, (h) adsorbents for example kaolin and bentonite, and (i) lubricants, for example talc, calcium stearate and magnesium stearate and solid polyethylene glycols, or mixtures of the compounds listed under (a) to (i).

The tablets, dragees, capsules and pills can be provided with the customary coatings and shells, optionally containing pacifying agents, and can also be of such composition that they release the active compound or compounds only, or preferably, in a certain part of the intestinal tract, optionally in a delayed manner, examples of embedding compositions which can be used being polymeric substances and waxes.

The active compound or compounds, optionally together with one or more of the above mentioned excipients could also be in a micro-encapsulated form.

Solutions and emulsions for parenteral administration can contain, in addition to the active compound or compounds, the customary excipients, such as solvents, solubilizing agents and emulsifiers, for example water, ethyl alcohol, isopropyl alcohol, ethyl carbonate, ethyl acetate, benzyl alcohol, benzyl benzoate, propylene glycol, 1,3-butylene glycol, dimethylformamide, oils, especially cotton seed oil, groundnut oil, maize germ oil, olive oil, castor oil and sesame oil, glycerol, glycerol-formal, tetrahydrofurfuryl alcohol, polyethylene glycol and fatty acid esters of sorbitol, or mixtures of these substances, in a sterile form which is isotonic with blood.

The therapeutically active compounds should preferably be present in the above-mentioned pharmaceutical formulations in a concentration of about 0.1 to 99.5, preferably of about 0.5 to 95% by weight of the total mixture.

The above-mentioned pharmaceutical formulations can also contain other pharmaceutical formulations and/or other pharmaceutical active compounds in addition to the active compounds according to the invention.

The above-mentioned pharmaceutical formulations are prepared in the customary manner according to known methods, for example by mixing the active compound or compounds with the excipient or excipients.

The present invention also includes compounds according to the invention, and of pharmaceutical formulations which contain one or more active compounds according to the invention for use in human and veterinary medicine.

The actual dosage unit will be determined by such generally recognized factors as body weight of the patient and/or severity and type of pathological condition the patient might be suffering. With these considerations in mind, the dosage unit for a particular patient can be readily determined by the medical practitioner in accordance with the techniques known in the medical arts.

The precise instructions for pharmaceutical administration of the compounds and agents according to the invention necessarily depend on the requirements of the individual case, the nature of treatment, and of course the opinion of the treating physician.

### Examples

### Reference Example 1

### 2-(3-Benzyl-6,7-dimethoxy-4-oxo-3,4-dihydro-quinazolin-2-yl-thio)acetohydrazide (HHG-70)

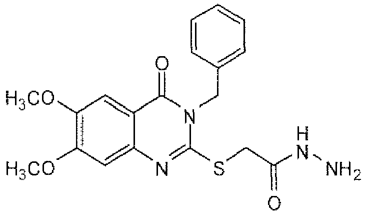

To 4.1 g (0.01 mol) of Ethyl 2-(3-benzyl-6,7-dimethoxy-4-oxo-3,4-dihydro-quinazolin-2-yl-thio)acetate, 5.0 ml (0.02 mol) of hydrazine hydrate were added in 50 ml of absolute ethanol and the mixture was stirred at room temperature. Stirring was continued at room temperature for several days and the product formed was filtered, triturated with petroleum ether (40°-60°). The solid obtained was washed, dried and recrystallized from chloroform to give the required product (3.15 g, 79% yield), mp 170-2°C, m/e 401, 100% (consistent with molecular formula C₁₉H₂₀N₄O₄S, calcd. 400.45). ¹H-NMR (DMSO-d₆): δ 3.9 (m, 8H, SCH₂ and OCH₃), 4.31 (s, 2H, Exchange.NH₂), 5.30 (s, 2H, PhCH₂), 7.01 (m, 1H, ArH), 7.20-7.35 (m, 5H, ArH), 7.45 (m, 1H, ArH), 9.35 (s, 1H, Exchange.NH). ¹³C-NMR: δ 34.0, 39.5, 46.7, 55.7, 56.0, 62.9, 105.5, 106.9, 111.5, 126.7, 127.4, 128.6, 135.8, 143.1, 148.1, 154.2, 154.9, 160.2, 166.1.

### Example 2

### N'-[2-(6-Chloro-4-oxo-3-phenyl-3,4-dihydro-quinazolin-2yl-thio)acetyl]propionohydrazide (HHG-89)

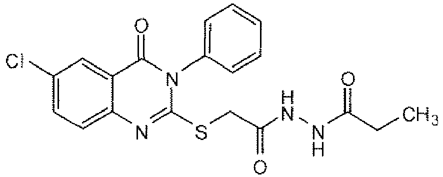

A mixture of 2-(6-chloro-4-oxo-3-phenyl-3,4-dihydro-quinazolin-2-yl-thio)acetohydrazidc (3.6 g, 0.01 mol), propionyl chloride (1.9 g, 0.02 mol) and catalytic amount of dry pyridine in dry chloroform was stirred at room temperature for 2-3 hours. The obtained product was filtered, dried, triturated with petroleum ether (40°-60°) and the separated solid was washed with distilled water and recrystallized from methanol to give a white powder (2.3g, 55% yield), mp 128-2°C, m/e 417, 100% (consistent with molecular formula C₁₉H₁₇ClN₄O₃S, calcd. 416.88). ¹H-NMR (DMSO-d₆): δ 1.00 (t, 3H, J=14.5 Hz, CH₂CH₃), 2.12 (q, 2H, J=7.5, 14.5 Hz, COCH₂CH₃), 3.90 (s, 2H, SCH₂), 7.48 (m, 2H, ArH), 7.62 (m, 3H, ArH), 7.72 (d, 1H, *J*=9 Hz, ArH), 7.90 (dd, 1H, *J*=2, 9 Hz, ArH), 8.20 (d, 1H, *J*=3 Hz, ArH), 9.85 (s, 1H, NH), 10.15 (s, 1H, NH). ¹³C-NMR: δ 10.2, 26.9, 34.9, 39.6, 39.8, 40.3, 40.5, 40.7, 121.4, 125.9, 129.1, 129.8, 130.1, 130.7, 135.4, 136.1, 146.4, 166.1, 172.2.

### Example 3

### N'-Acetyl-2-(3-benzyl-6-chloro-4-oxo-3,4-dihydro-quinazolin-2-yl-thio)acetohydrazide (HGG-105)

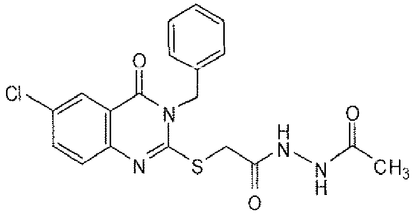

A mixture of 2-3-(benzyl-6-chloro-4-oxo-3,4-dihydro-quinazolin-2-yl-thio)acetohydrazide (3.8 g, 0.01 mol), acetyl chloride (1.6 g, 0.02 mol) and catalytic amount of dry pyridine in dry chloroform was stirred at room temperature for 2-3 hours. The product produced was filtered, dried , triturated with petroleum ether (40°-60°) and the separated solid was washed with distilled water and recrystallized from chloroform to give a white powder (3.0 g, 73% yield), mp 206-7°C, m/e 417, 100% (consistent with molecular formula C₁₉H₁₇ClN₄O₃S, calcd. 416.88). IR (cm⁻¹); 1688 (C=O str.), 3153-3349 (N-H str.). ¹H-NMR (DMSO-d₆): δ 1.85 (s, 3H, CH₃), 4.00 (s, 2H, SCH₂), 5.35 (s, 2H, PhCH₂), 7.25-7.37 (m, 6H, ArH and NH), 7.70 (d, 1H, *J*=9 Hz, ArH), 7.87 (dd, 1H, *J*=2, 8.5 Hz, ArH), 8.50 (d, 1H, *J*=3 Hz, ArH), 10.00 (brs, 1H, NH). ¹³C-NMR: δ 20.9, 34.6, 39.8, 40.27, 40.4, 47.7, 120.4, 125.9, 127.3, 128.1, 129.2, 130.7, 135.4, 135.8, 146, 157.5, 160.5, 166.5, 168.3.

### Example 4

### N'-(2-(3-Benzyl-6,7-dimethoxy-4-oxo-3,4-dihydro-quinazolin-2-yl-thio)acetyl)propionohydrazide (HHG-110)

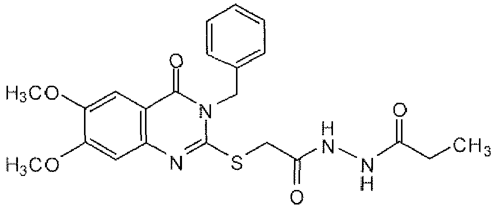

A mixture of 2-(3-benzyl-6,7-dimethoxy-4-oxo-3,4-dihydro-quinazolin-2-yl-thio)acetohydrazide (4.0 g, 0.01 mol), propionyl chloride (1.9 g, 0.02 mol) and catalytic amount of dry pyridine in dry chloroform was stirred at room temperature for 2-3 hours. The product produced was filtered, dried, triturated with petroleum ether (40°-60°) and the separated solid was washed with distilled water and recrystallized from ethanol/chloroform mixture to give a white powder (4.4 g, 96% yield), mp 197-9°C, m/e 457, 100% (consistent with molecular formula C₂₂H₂₄N₄O₅S, calcd. 456.15). ¹H-NMR (DMSO-d₆): δ 1.00 (t, 3H, *J*=15 Hz, CH₃), 2.15 (q, 2H, COCH₂), 3.85 (s, 3H, OCH₃), 3.95 (s, 3H, OCH₃), 4.00 (s, 2H, SCH₂), 5.32 (s, 2H, PhCH₂), 7.20-7.30 (m, 4H, ArH), 7.35 (d, 2H, *J*=7 Hz, ArH), 7.40 (s, 1H, ArH), 9.90 (s, 1H, NH), 10.25 (s, 1H, NH). ¹³C-NMR: δ 9.5, 26.2, 33.8, 39.2, 39.7, 46.7, 55.6, 55.9, 105.3, 107.4, 111.4, 126.7, 127.4, 128.5, 135.7, 143.2, 148.1, 154, 154.9, 160.2, 165.6, 171.5.

### Example 5

### N'-(2-(3-Benzyl-6-chloro-4-oxo-3,4-dihydro-quinazolin-2-yl-thio)acetyl)-3-phenylpropanehydrazide (HHG-115)

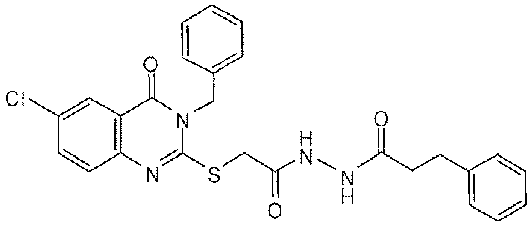

A mixture of 2-(3-benzyl-6-chloro-4-oxo-3,4-dihydro-quinazolin-2-yl-thio)acetohydrazid (3.8 g, 0.01 mol), 3-phenyl-propanoyl chloride (3.4 g, 0.02 mol) and catalytic amount of dry pyridine in dry chloroform was stirred at room temperature for 2-3 hours. The product produced was filtered, dried , triturated with petroleum ether (40°-60°) and the separated solid was washed with distilled water and recrystallized from chloroform to give a white powder (3.5 g, 68% yield), mp 199-201°C, m/e 507, 100% (consistent with molecular formula C₂₆H₂₃ClN₄O₃S, calcd. 507.00). IR (cm⁻¹), 1686 (3x C=O str.), 3206 (N-H str.). ¹H-NMR (DMSO-d₆): δ 2.45 (t, 2H, *J*=15.5 Hz,COCH₂CH₂), 2.80 (t, 2H, *J*=15.5 Hz, COCH₂), 4.05 (s, 2H, SCH₂), 5.35 (s, 2H, PhCH₂), 7.14-7.38 (m, 10H, ArH), 7.69 (d, 1H, *J*=9 Hz, ArH), 7.87 (dd, 1H; *J*=2, 9 Hz, ArH), 8.05 (d, 1H, *J*=2 Hz, ArH), 9.90-10.40 (s, 2H, NHs). ¹³C-NMR: δ 31.2, 34.6, 35.2, 39.6, 39.8, 40, 40.3, 40.5, 47.7, 120.4, 125.9, 126.4, 127.3, 128.1, 128.7, 128.8, 129.1, 130.7, 135.5, 135.8, 141.5, 146.1, 157.5, 160.5, 165.9, 170.5.

The NMR spectral data assignments of compounds of Example 1-5 are based on analysis of the ¹H, Attached Proton Test (APT). The Distortionless Enhancement Polarization Transfer (DEPT), correlated spectroscopy (COSY), Heteronuclear Multiple Quantum Coherence Spectroscopy (HMQC), NMR spectra for each compound .

Adult male Swiss amino mice (22-28 g), approximately 10-week old, were used to conduct the anticonvulsant evaluation. They were housed in cages and kept at a temperature of 20 ± 2°C and a relative humidity of 55 ± 5% with a light-dark cycle of 12 h and fed with standard diet and water *ad libitum.* Compounds of the formula 1 have been dissolved in dimethylsulfoxide and administered at dose levels of 25, 50, 100 and 200 mg/kg intraperitoneal (i.p.). Sodium valproate was freshly dissolved in 0.9 % NaCl and used at dose level of 200 mg/kg (1.38 mmol/kg) i.p., 30 min after the test compounds as a positive control. A control group of mice was included and received intraperitoneal (i.p.) injections of the test compounds' vehicle. Pentylenetetrazole (PTZ) was freshly dissolved in 0.9% NaCl and used at dose level of 100 mg/kg, i.p. This dose was found to be the minimum dose that induced 100% clonic convulsion. Picrotoxin (pic) was freshly dissolved in 1 ml 0.1N warmed HCl and the final volume was made up with 0.9% NaCl. It has been used at dose level of 10 mg/kg i.p. and was given 30 min after the test compounds.

The anticonvulsant activity of compounds of the formula 1 in mice was measured using Pentylenetetrazole (PTZ) seizure threshold test (White, H.S. et al. In Antiepileptic Drugs, 4th ed.; Levy, R.H. et al., Eds, Raven: New York, 1995, pp 99-121), The active compounds of the formula 1 were further tested against maximal electroshock seizure (MES), and picrotoxin induced clonic convulsions, to explore the involvement of GABA-ergic receptors in their anticonvulsant activity. The effect of compounds of the formula 1 on motor performance was studied by chimney test (Dauge, V. et al. Neuropsychopharmacology 2001, 25, 690-98). Median effective (ED₅₀), and median sedative (TD₅₀) were calculated. Representative examples are shown in Table 1, Examples 6-10.

The acute toxicity detenninatioii (LD₅₀) for compounds of formula 1 was performed. Compounds were given intraperitoneally (i.p.) in doses ranging from 0.1-5 mmol/kg. The animals were observed for up to 6 hours continuously and were then kept under observation for 72 hours. All behavioral changes and death during the observation periods were recorded. The percentage of death at each dose level was then calculated, and the LD₅₀ values were obtained (Ghosh, M., Fundamentals of Experimental Pharmacology, Scientific Book Agency, Calcutta. 1984, pp 153-158, 187-189). The protective index (PI) and therapeutic index (TI) of each compound were calculated. Representative examples are shown in Table 1, Examples 11-12.

Biological evaluation of the new compounds of the formula 1 of the invention revealed that the compounds possess a remarkable anticonvulsant activity. The obtained results clearly point to the discovery of a new group of anticonvulsant agents that induce their actions via interaction with GABA_{A} receptors. The safety of the new compounds of the formula 1 of the invention is comparable to that of valproate sodium. Therefore, compounds of the formula 1 of the invention have the potential use as anticonvulsants, with potency proved to he 3-10 fold more active than valproate sodium.

### Example 6

### Effect of HHG-70, HHG-89, HHG-105, HHG-110 and HHG-115 on Pentylenetetrazole (PTZ)-induced convulsion

Pentylenetetrazole (PTZ) seizure threshold test is used to evaluate the potential anticonvulsant activity of compounds of the formula 1 (White, H.S. et al. In Antiepileptic Drugs, 4th ed.; Levy, R.H. et al., Eds, Raven: New York, 1995, pp 99-121). Each compound was tested in 4 mice groups (Swiss albino mice, 25 g body weight) for each dose level (25, 50, 100 and 200 mg/kg) and was given i.p. 30 min later, animals were received PTZ (100 mg/kg, i.p.) and observed for 30 min. A single 5 seconds episode of clonic spasms was taken as a threshold seizure. HHG-70, HHG-89, HHG-105, HHG-110 and HHG-115 produced 100% protection against PTZ-induced seizures, Table 1.

**Table 1: ED₅₀, TD₅₀, PI, LD₅₀ and TI for HHG-70, HHG-89, HHG-105, HHG-110 and HHG-115**

| **Compound^{a}** | **Dose** | | **% Protection** | **ED₅₀^{b}** | **TD₅₀^{b}** | **PI^{c}** | **LD₅₀** | **TI^{d}** |
|---|---|---|---|---|---|---|---|---|
| | **mmole/Kg** | **mg/Kg** | | **mg/Kg** | **mg/Kg** | | **mg/Kg** | |
| **70** | 0.12 | 50 | 100 | 32.0 | 149.4 | 4.67 | 360.0 | 11.25 |
| **89** | 0.12 | 50 | 100 | 11.79 | 100.3 | 8.5 | 290.3 | 24.6 |
| **105** | 0.36 | 150 | 100 | 24.75 | 215.3 | 8.7 | 239.6 | 9.7 |
| **110** | 0.33 | 150 | 100 | 32.7 | 134.1 | 4.1 | 167.5 | 5.1 |
| **115** | 0.29 | 150 | 100 | 29.5 | 135.7 | 4.6 | 290.3 | 9.8 |
| **Sodium Valproate** | 1.13 | 50 | 100 | 189 | 470 | 2.4 | 985 | 5.2 |

| | | | | | | | | |
|---|---|---|---|---|---|---|---|---|
| ^{a} Each dose of selected compounds was tested using 4 animals and the percentage of animals protected was recorded and the anticonvulsant activity was calculated. ^{b} ED₅₀ and TD₅₀: Median effective and median sedative doses, respectively. ^{c} PI: Protective index = TD₅₀/ED₅₀ ^{d}TI: Therapeutic index = LD₅₀/ED₅₀. | | | | | | | | |

### Example 7

### Effect of HHG-70, HHG-89, HHG-105, HHIG-110 and HHG-115 against the maximal electroshock (MES)-induced convulsion

The maximal electroshock (MES) seizure is one of the electrical test used to evaluate anticonvulsant activity (White, H.S. et al. In Antiepileptic Drugs, 4th ed.; Levy, R.H. et al., Eds, Raven New York, 1995, pp 99-121). MES that induced 100% maximal seizure was found to be 50 mA alternating current of 100 Hz frequency for 0.2 seconds, using ECT UNIT (model number 7801, UGO Basile, Varese, Italy). Swiss albino mice (25 g body weight) were injected with 25, 50, 100 and 200 mg/kg of HHG-70, HHG-89, HHG-1 05, HHG-110 and HHG-115 i.p. 30 min later, mice were restrained by hand and subjected to electric shock through their ears, and released immediately following electrical stimulation, to permit observation of the maximal seizure. The maximal seizure typically consists of a short period of initial toxic flexioni and a prolonged period of tonic extension (especially the hind limb). Protection was defined as complete absence of hind limb tonic extension.

### Example 8

### Effect of HHG-70, HHG-89, HHG-105, HIIG-110 and HHG-115 against picrotoxin (pic)-induced convulsion

Swiss albino mice (25 g body weight) were injected with 25, 50, 100 and 200 mg/kg of HHG-70, HHG-89, HHG-105, HHG-110 and HHG-115 (4 mice, i.p.). 30 min later, animals were injected sc with pic (10 mg/kg) and observed for 30 min and the percentage protection against convulsive seizures for each compound was calculated.

### Example 9

### Minimal neurotoxicity (chimney test) for HHG-70, HHG-89, HHG-105, HHG-110 and HHG-115

The effect of HHG-70, HHG-89, HHG-105, HHG-110 and HHG-115 on motor performance was studied by chimney test (Dauge, V. et al. Neuropsychopharmacology 2001, 25, 690-98). At 15 and 25 min after i.p. administration of compounds HHG-70, HHG-89, HHG-105, HHG-110 and HHG-115, the inability of mice to climb up backwards in a glass tube of 25 cm length and 3 cm inner diameter within 30 sec was recorded and taken as a measure of neurological deficits. Normal mice climb up in 5-10 sec. The dose that impaired the motor coordination in 50% of the animals tested was labelled as the median toxic dose 50 (TD₅₀).

### Example 10

### Calculation of the anticonvulsant ED₅₀ values for HHG-70, HHG-89, HHG-105, HHG-110 and HHG-115

In each of the chemical and the electrical methods described above, Swiss albino mice were divided into 4 groups (N = 4 animals). Each group was injected (i.p.) with one of the four selected doses that have proven to obtain a variable percentage of protection 20 minutes before the convulsants. The effective dose 50 (ED₅₀) i.e. the dose that protected half of the animals was then calculated using the log-probit method (White, H.S. et al. In Antiepileptic Drugs, 4th ed.; Levy, R.H. et al., Eds, Raven: New York, 1995, pp 99-121).

### Example 11

### Determination of LD₅₀ and dose-response curve:

Initially the approximate LD₅₀ of each test compound was determined. Albino Swiss mice (25 g body weight) were then divided into 4 groups (6 mice each). Each group was then injected with one of 4 selected doses the highest being the LD₁₀₀ dose. The animals were then observed closely for 4 hours and then occasionally for 72 hours. The percentage of death in each group was then calculated. The LD₅₀ doses were then calculated using the Probit method (Ghosh; M., Fundamentals of Experimental Pharmacology, Scientific Book Agency, Calcutta. 1984, pp 153-158, 187-189).

### Example 12

### Calculation of the Protective Index (PI) and Therapeutic Index for HHG-70, HHG-89, HHG-105, HHG-110 and HHG-115

The protective index (PI) - i.e. the value that indicates the margin of safety and tolerance of the test compound was calculated by the formula: Protective index (PI) = TD₅₀/ED₅₀. The Therapeutic Index was calculated by the formula: Therapeutic index (TI) = LD₅₀/ED₅₀ (Löscher, W. et al. Epilepsy Res. 1991, 9, 1-10).

## Claims

1. Compound according to formula 1: 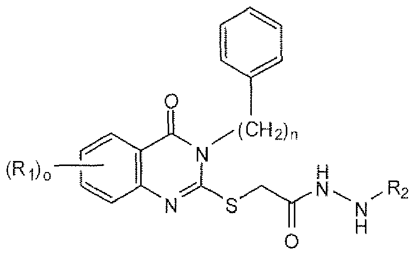
wherein each R₁ is independently selected from the group consisting of chloro, methoxy, aryl, heteroaryl, mercapto and alkylthio;
R₂ is -CO-(CH₂)ₘ-R₃;
R₃ is selected from the group consisting of alkyl, alkoxyl, aryl, or heteroaryl ring system;
o is an integer from 1-4; and
n and m are independently selected integers form 0-10.

2. Compounds according to claim 1, wherein R₁ is phenyl or naphthyl.

3. Compound according to claim 1 or 2, wherein heteroaryl is furyl, pyrrolyl, thienyl, imidazolyl, thiadiazolyl, pyridinyl, pyridazinyl, pyrimidinyl, benzothiazolyl or oxadiazolyl.

4. Compound according to any of the preceding claims, wherein R₁ is independently selected from the group consisting of chloro or methoxy.

5. Compound according to any of the preceding claims, wherein n and m are independently selected integers from 0 - 5.

6. Method for preparing the compound according to any of the claims 1to 5, according to the following reaction sequence: 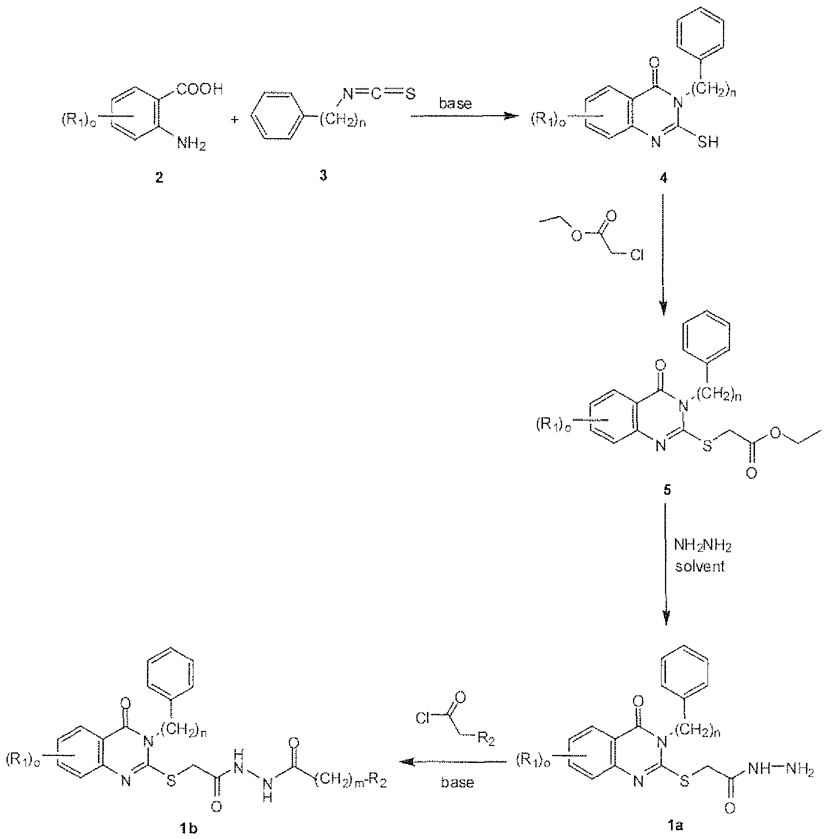 wherein R₁, R₂, n, m and o are defined as in claims 1 to 5.

7. Pharmaceutical composition comprising at least one compound according to any of the claims 1 to 5.

8. Compound according to any of the claims 1 to 5 for use in the treatment of convulsion and/or epilepsy.

9. Pharmaceutical composition according to claim 7 for use in the treatment of convulsion and/or epilepsy.

## Patentansprüche

1. Verbindung gemäß der Formel 1: 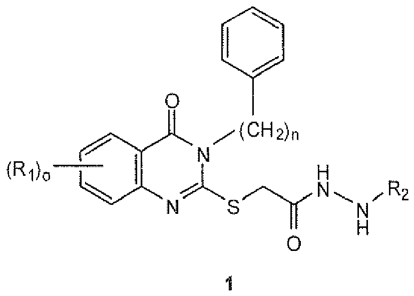
wobei jedes R₁ unabhängig aus der Gruppe ausgewählt ist, die aus Chlor, Methoxy, Aryl, Heteroaryl, Mercapto und Alkylthio besteht;
R₂ -CO-(CH₂)ₘ-R₃ ist;
R₃ aus der Gruppe ausgewählt ist, die aus Alkyl, Alkoxy, Aryl oder einem Heteroaryl-Ringsystem besteht;
o eine ganze Zahl von 1 bis 4 ist; und
n und m unabhängig ausgewählte ganze Zahlen von 0 bis 10 sind.

2. Verbindung nach Anspruch 1, wobei R₁ Phenyl oder Naphthyl ist.

3. Verbindung nach Anspruch 1 oder 2, wobei das Heteroaryl Furyl, Pyrrolyl, Thienyl, Imidazolyl, Thiadiazolyl, Pyridinyl, Pyridazinyl, Pyrimidinyl, Benzothiazolyl oder Oxadiazolyl ist.

4. Verbindung nach einem der vorangehenden Ansprüche, wobei R₁ unabhängig aus der Gruppe ausgewählt ist, die aus Chlor oder Methoxy besteht.

5. Verbindung nach einem der vorangehenden Ansprüche, wobei n und m unabhängig ausgewählte ganze Zahlen von 0 bis 5 sind.

6. Verfahren zur Herstellung der Verbindung nach einem der Ansprüche 1 bis 5 gemäß der folgenden Reaktionssequenz: 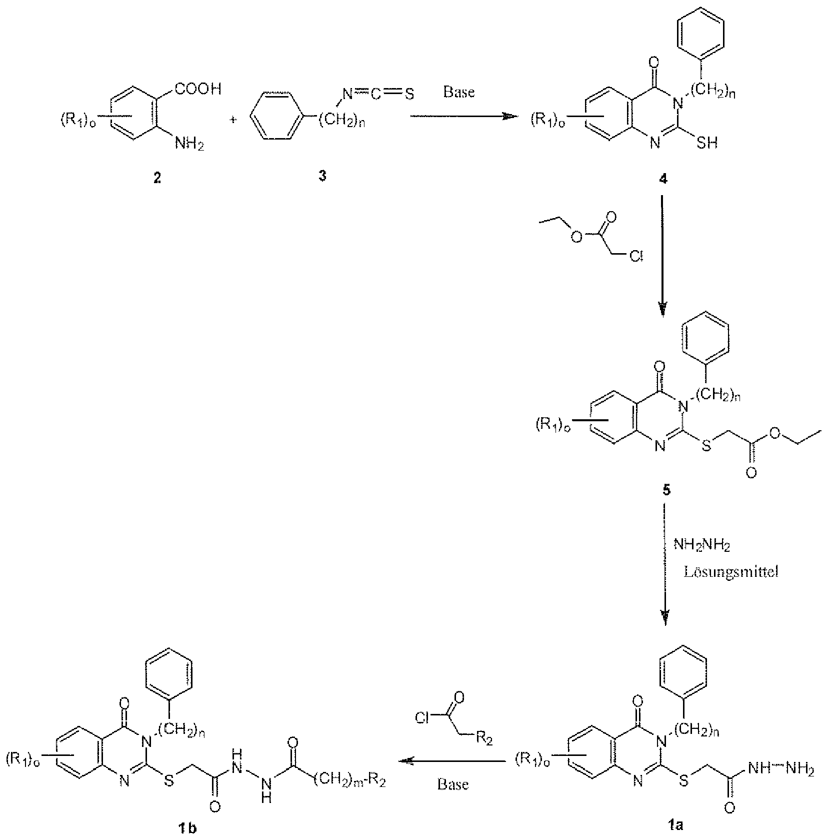 wobei R₁, R₂, n, m und o wie in den Ansprüchen 1 bis 5 definiert sind.

7. Pharmazeutische Zusammensetzung, die zumindest eine Verbindung nach einem der Ansprüche 1 bis 5 umfasst.

8. Verbindung nach einem der Ansprüche 1 bis 5 zur Verwendung in der Behandlung von Konvulsion und/oder Epilepsie.

9. Pharmazeutische Zusammensetzung nach Anspruch 7 zur Verwendung in der Behandlung von Konvulsion und/oder Epilepsie.

## Revendications

1. Composé selon la formule 1 : 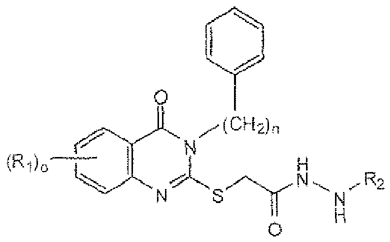
dans laquelle chaque R₁ est choisi indépendamment dans le groupe constitué par un chloro, méthoxy, aryle, hétéroaryle, mercapto et alkylthio ;
R₂ est -CO-(CH₂)ₘ-R₃ ;
R₃ est choisi dans le groupe constitué par un alkyle, alcoyle, aryle, ou un système de cycle hétéroaryle ;
o est un entier de 1 à 4 ; et
n et m sont des entiers choisi indépendamments entre 0 et 10.

2. Composé selon la revendication 1, dans lequel R₁ est phényle ou naphtyle.

3. Composé selon la revendication 1 ou 2, dans lequel l'hétéroaryle est un furyle, pyrrolyle, thiényle, imidazolyle, thiadiazolyle, pyridinyle, pyridazinyle, pyrimidinyle, benzothiazolyle ou oxadiazolyle.

4. Composé selon l'une quelconque des revendications précédentes, dans lequel R₁ est choisi indépendamment dans le groupe constitué par un chloro ou méthoxy.

5. Composé selon l'une quelconque des revendications précédentes, dans lequel n et m sont des entiers choisi indépendamments entre 0 et 5.

6. Procédé de préparation du composé selon l'une quelconque des revendications 1 à 5, selon la séquence de réaction suivante : 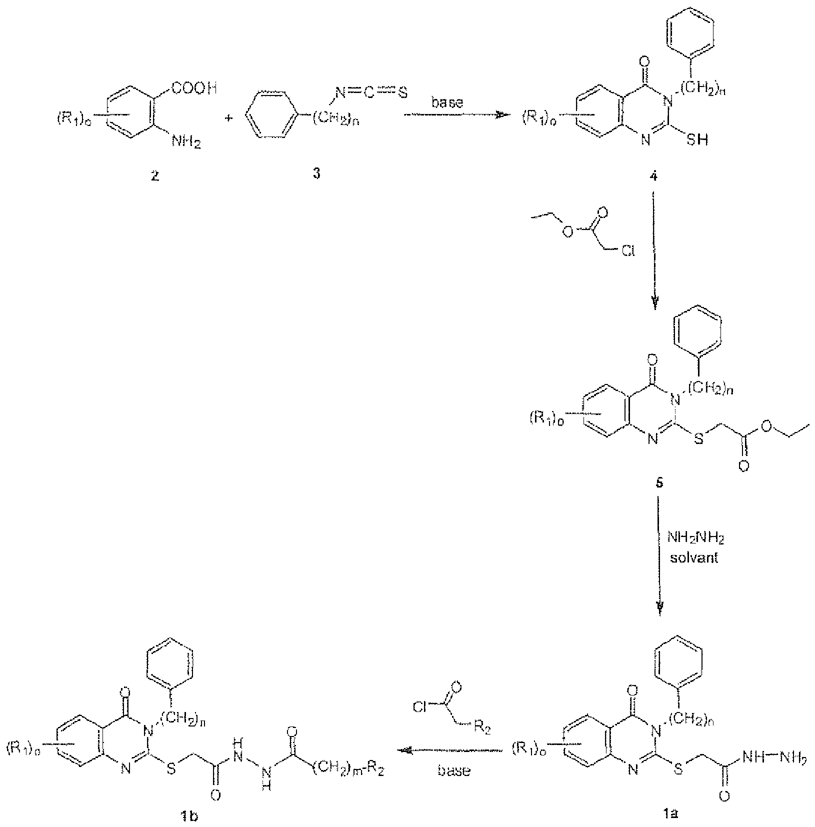 dans laquelle R₁, R₂, n, m et o sont tels que définis dans les revendications 1 à 5.

7. Composition pharmaceutique comprenant au moins un composé selon l'une quelconque des revendications 1 à 5.

8. Composé selon l'une quelconque des revendications 1 à 5 pouvant être utilisé dans le traitement des convulsions et/ou de l'épilepsie.

9. Composition pharmaceutique selon la revendication 7 pouvant être utilisée dans le traitement des convulsions et/ou de l'épilepsie.
